# EUROPÄISCHE PATENTSCHRIFT

(11) **EP 3 498 203 B1**
(45) Veröffentlichungstag und Bekanntmachung des Hinweises auf die Patenterteilung: **03.11.2021**
(21) Anmeldenummer: 17206694.6
(22) Anmeldetag: 12.12.2017
(51) Int. Cl.: A61B 17/34, A61B 18/14, A61B 17/00, A61B 18/00

(54) **SONDENAPPLIKATOR**
PROBE APPLICATOR
APPLICATEUR DE SONDE

(43) Veröffentlichungstag der Anmeldung: 19.06.2019
(73) Patentinhaber: Erbe Elektromedizin GmbH, 72072 Tübingen (DE)
(72) Erfinder: Rombach, Thorsten, 72810 Gomaringen (DE)
(74) Vertreter: Rüger Abel Patentanwälte PartGmbB

(56) Entgegenhaltungen:
- CN-U- 204 147 118
- DE-A1- 10 354 147
- US-A- 5 314 406
- US-A1- 2012 253 116
- US-A1- 2014 236 147

## Beschreibung

Gegenstand der Erfindung ist ein Applikator zum laparoskopischen Einsatz von flexiblen Sonden.

Aus der US 5,314,406 ist ein medizinisches Instrument mit einem länglichen Schaft bekannt, dessen proximales Ende an einem Gehäuse mit Handgriff gehalten ist. An der dem Schaft gegenüber liegenden Seite des Gehäuses ist eine Einführöffnung vorgesehen, durch die eine Sonde durch das Gehäuse in den Schaft eingeschoben werden kann. Die Sonde durchragt den Schaft und kann am distalen Ende desselben aus diesem herausragen. Der Schaft dient dann der Absaugung von Fluiden vom Behandlungsort, was über entsprechende Ventile an dem Griff gesteuert werden kann. An dem Griff des Gehäuses sind Anschlüsse für Fluidleitungen zum Spülen und Absaugen angeordnet. Die Sonde ist ihrerseits an eine elektrische Quelle angeschlossen.

Davon ausgehend ist es Aufgabe der Erfindung, einen Sondenapplikator bereitzustellen, der den Einsatz flexibler Sonden außerhalb von endoskopischen Anwendungen gestattet.

Diese Aufgabe wird mit dem Sondenapplikator nach Anspruch 1 gelöst.
Die vorliegende Erfindung wird im Hauptanspruch 1 definiert.
Die bevorzugten Ausführungsformen werden in den Nebenansprüchen definiert.

CN 204 147 118 U offenbart ein Instrument mit Ablationselektrode.
US 5 314 406 A offenbart ein elektrochirurgisches Instrument zur Aufnahme von Sonden.
DE 103 54 147 A1 offenbart eine medizinische
Steckverbindungsvorrichtung mit einem Stecker und einer Kupplung zum Anschließen einer elektroakustischen Therapiequelle an eine Versorgungseinheit.
US 2014/236147 A1 offenbart einen Schaft für ein starres endoskopisches Instrument.
US 2012/253116 A1 offenbart ein chirurgisches System mit wiederverwendbarem Griff.

Der erfindungsgemäße Sondenapplikator weist ein Gehäuse mit einem Schaft auf, der ein längliches steifes Rohr ist, dessen proximales Ende mit dem Gehäuse verbunden ist. Der Schaft ist an seinen beiden Enden offen. Der Schaft ist wesentlich steifer als die durch ihn hindurch zu schiebenden Sonden. Die Steifigkeit des Schafts ist vorzugsweise wenigstens so groß, dass eine an dem distalen Ende des Schafts von z.B. 200 mm bis 400 mm Länge angreifende Querkraft von 0,5 N eine seitliche elastische Auslenkung des Schafts bewirkt, die nicht größer als 75 mm ist. Bedarfsweise kann der Schaft steifer sein, so dass z.B. bei der besagten Länge und der genannten Kraft lediglich eine Auslenkung von 6 mm oder weniger auftritt.

An der dem Schaft gegenüber liegenden Seite des Gehäuses ist eine Sonden-Einführöffnung vorgesehen, durch die eine flexible Sonde in das Gehäuse und durch dieses hindurch in den Schaft eingeschoben werden kann. Das mit der Sonde bestückte Gehäuse kann dann mittels des an dem Gehäuse vorgesehenen Griffs manuell positioniert und bewegt werden, um den Schaft zum Beispiel durch eine kleine Zugangsöffnung in den Körper des Patienten einzuführen und dort durch Aktivierung der Sonde und Bewegung des Gehäuses mit dem Schaft gewünschte operative Eingriffe vorzunehmen. Dabei sind ein oder mehrere Steuerelemente zur Aktivierung und Deaktivierung der Sonde an dem Gehäuse, insbesondere an dessen Griff angeordnet.

Von dem Gehäuse erstreckt sich ein Kabel zu einem Stecker, um von einer Betätigung des Steuerelements ausgehende Signal an einen Stecker oder sonstigen Verbinder zu übertragen. Die sich durch das Kabel erstreckende Signalleitung kann eine elektrische Leitung, eine Fluidleitung, ein Wellenleiter, wie zum Beispiel eine optische Faser oder dergleichen, sein.

Zusätzlich weist der Sondenapplikator eine Anschlussvorrichtung auf, die zum Anschluss einer Sonde eingerichtet ist, um diese mit Betriebsmedium zu versorgen. Das Betriebsmedium kann elektrischer Strom, ein flüssiges oder gasförmiges Fluid, Lichtenergie oder dergleichen sein. Vorzugsweise ist die Anschlussvorrichtung an dem Stecker vorgesehen, über den der Sondenapplikator mit einem Versorgungsgerät zu verbinden ist. Alternativ kann die Anschlussvorrichtung auch von dem Stecker gesondert ausgebildet sein und beispielsweise durch eine Buchsenanordnung gebildet sein, die über eine flexible Leitung mit dem Stecker verbunden ist. Die Anschlussvorrichtung kann auch an dem Gehäuse angeordnet sein.

Der erfindungsgemäße Sondenapplikator gestattet den Einsatz einer Vielzahl von aus der Endoskopie bekannten flexiblen Sonden außerhalb von Endoskopen. Beispielsweise kann der Nutzer den Sondenapplikator wie bei laparoskopischen Eingriffen einsetzen. Damit werden endoskopische Sonden für chirurgische Disziplinen in der Allgemeinchirurgie, in der Viszeralchirurgie, in der Gynäkologie, der Urologie und im Bereich Hals-Nasen-Ohren (HNO) nutzbar gemacht.

Der starre Schaft des Sondenapplikators stabilisiert flexible Sonden bis über eine Länge von bis zu 45 oder mehr Zentimetern. Damit können in der Laparoskopie und der Mini-Laparoskopie flexible Sonden genutzt werden, ohne gesonderte starre Instrumente mit gleicher Funktion bereitstellen zu müssen.

Weiter eröffnet der erfindungsgemäße Sondenapplikator die Bedienung der Sonden insbesondere im Wege der Handaktivierung, was bei chirurgischen Anwendungen ein wichtiger Aspekt ist. Der Chirurg kann sowohl die Positionierung, Bewegung und Führung, als auch die Aktivierung und Deaktivierung der Sonde mit einer Hand an dem Gehäuse, d.h. an dem Handgriff und den daran vorhandenen Steuerelementen vornehmen.

Der Schaft ist schlank und lang, wobei sein Durchmesser vorzugsweise höchstens 5 mm und bei einigen Anwendungsfällen höchstens 3 mm beträgt. Die Länge des Schafts übersteigt vorzugsweise wenigstens 100 mm weiter vorzugsweise wenigstens 350 mm oder 450 mm. Der Schaft ist steif, insbesondere druck- und biegesteif, um sein distales Ende im Körper des Patienten durch Bewegung des Griffs und Gehäuses positionieren und gezielt bewegen zu können.

Bei einer bevorzugten Ausführungsform ist an dem distalen Ende des Schafts eine Abwinkelungseinrichtung angeordnet, die zum Beispiel durch einen flexiblen rohrförmigen Abschnitt, einen balgartigen Abschnitt oder dergleichen gebildet ist, der von einer gestreckten Form in eine Bogenform überführbar ist. Dabei genügt es, wenn die Abwinkelungseinrichtung eine Schwenkbewegung um eine einzige Achse ausführen kann. Dabei krümmt sich der flexible Abschnitt um eine quer zu dem Schaft verlaufende, diesen nicht schneidende Krümmungsachse. Die Abwinkelungseinrichtung hat vorzugsweise lediglich einen Bewegungsfreiheitsgrad, nämlich einen Schwenkbewegungsfreiheitsgrad. Der Schaft kann einen zusätzlichen Freiheitsgrad, nämlich Drehung um seine Längsachse haben.

[1:1011] Zur Orientierung eines abgewinkelten Endes des Schafts in verschiedene Raumrichtungen kann der Schaft durch Bewegung des Gehäuses oder alternativ durch Drehung des Schafts an dem Gehäuse um seine Längsrichtung gedreht werden. Damit kann der Operateur das aus dem Schaft herausschauende Ende der Sonde auf einem Kegelmantel bewegen, wobei der Öffnungswinkel dieses Kegels durch Verstellung der Abwinkelungseinrichtung variabel ist.

Die Sondeneinführöffnung kann außerdem mit einer Feststelleinrichtung zur axialen Fixierung der Sonde versehen sein, so dass der Operateur die Sonde aus dem Schaft distal heraus schieben oder in diesen zurückziehen und diese Position dann fixieren kann.

Weitere Einzelheiten vorteilhafter Ausführungsformen der Erfindung sind Gegenstand der Zeichnung, der Beschreibung oder von Ansprüchen. In der Zeichnung sind Ausführungsbeispiele der Erfindung veranschaulicht. Es zeigen:
Figur 1 einen Sondenapplikator mit Sonde, in vereinfachter perspektivischer Darstellung,
Figur 2 den Sondenapplikator nach Figur 1 ohne Sonde, in vereinfachter perspektivischer Darstellung,
Figur 3 den Sondenapplikator mit Sonde, in einer schematisierten Seitenansicht,
Figur 4 eine an dem Schaft des Instruments vorgesehene Abwinkeleinrichtung in vergrößerter schematisierter Darstellung,
Figur 5 den Stecker des Sondenapplikators mit zwei verschiedenen, in den Stecker einsetzbaren Sonden und
Figur 6 eine abgewandelte Ausführungsform eines Steckers und der für die Sonden vorgesehenen Anschlussvorrichtung, in schematisierter Seitenansicht.

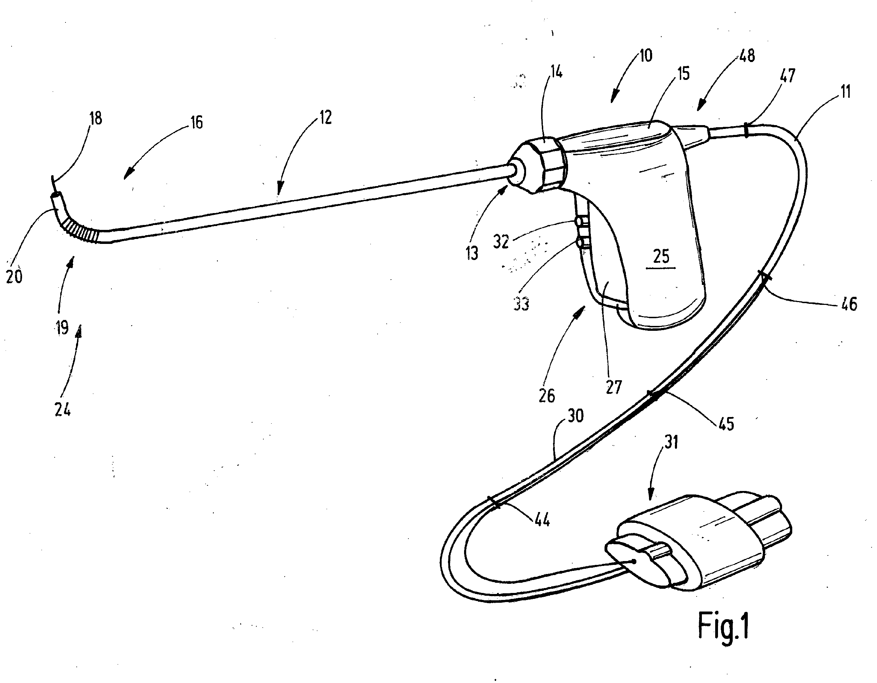

In Figur 1 ist ein Sondenapplikator 10 veranschaulicht, der zur Aufnahme von flexiblen Sonden, wie beispielsweise der Sonde 11, eingerichtet ist, um mit dieser Sonde 11 laparoskopische Eingriffe durchführen zu können. Dazu weist der Sondenapplikator 10 nach Art eines laparoskopischen Instruments einen länglichen Schaft 12 auf, dessen proximales Ende 13 in oder an einer Schaftaufnahme 14 gehalten ist. Vorzugsweise ist die Schaftaufnahme 14 um die Längsachse des Schafts 12 drehbar, sie kann jedoch auch starr, d.h. bezüglich eines Gehäuses 15 unverdrehbar ausgebildet oder angeordnet sein.

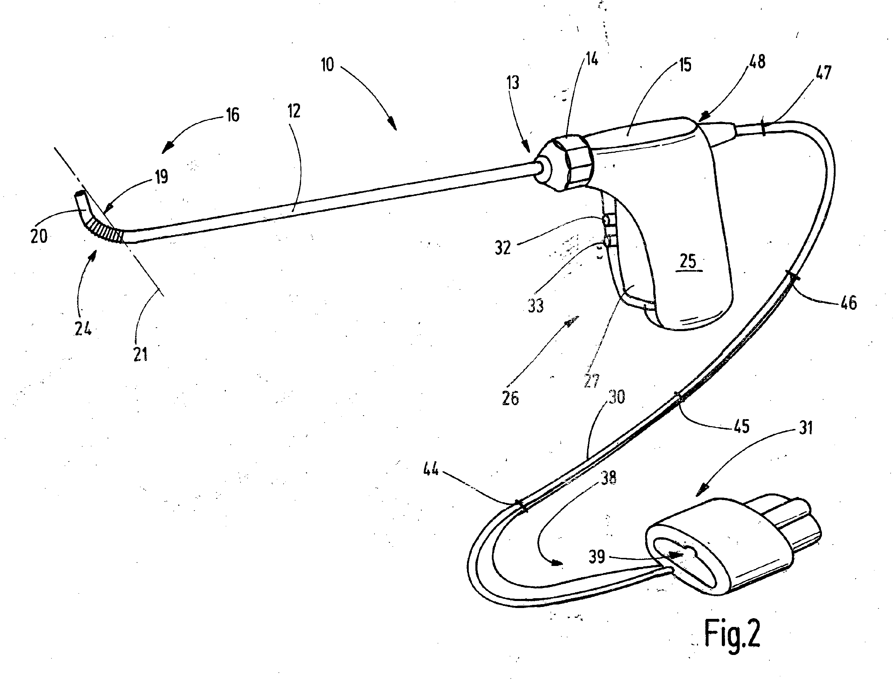

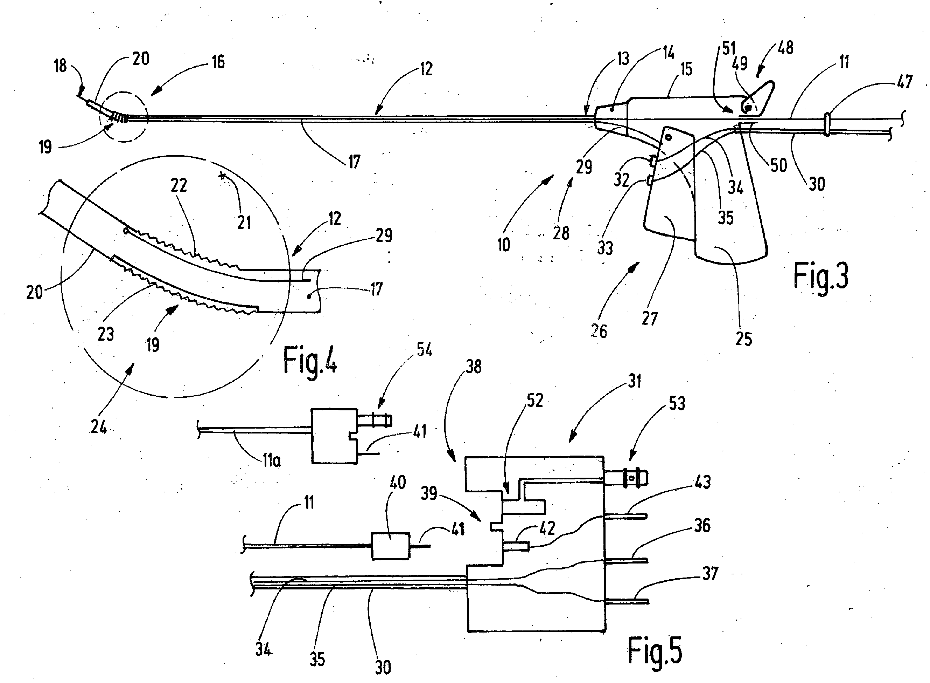

Figur 2 veranschaulicht den Sondenapplikator 10 ohne Sonde 11. Zur besseren Verdeutlichung des prinzipiellen Aufbaus des Sondenapplikators 10 ist dieser in Figur 3 nochmals mit Sonde 11 in einer schematischen Prinzipdarstellung veranschaulicht.

Der Schaft 12 wird durch ein schlankes Rohr gebildet, dessen Durchmesser vorzugsweise 5 mm nicht überschreitet und dessen Länge vorzugsweise größer ist als 200 mm. Die Schaftlänge kann bis zu 500 mm und in Sonderfällen auch mehr betragen. Das Rohr, aus dem der Schaft 12 ausgebildet ist, kann aus Metall oder Kunststoff bestehen und ist vorzugsweise so steif, dass es, wenn es durch eine kleine Öffnung in den Körper eines Patienten eingeführt wird, durch Bewegung seines proximalen Endes 13 im Körperinneren positioniert und bewegt werden kann, so dass das distale Ende 16 des Schafts 12 gewünschte Positionen einnimmt oder Bewegungen ausführt. Jedenfalls ist der Schaft 12 wesentlich steifer als die im Vergleich dazu flexible Sonde 11, die typischerweise für den endoskopischen Einsatz vorgesehen ist und die sich durch ein längs durchgehendes Lumen 17 des Schafts 12 erstreckt, so dass sie mit einem Ende 18 aus dem Schaft 12 herausragen kann.

Der Schaft 12 kann einen flexiblen Abschnitt 19 aufweisen, der in Figur 4 gesondert veranschaulicht ist. Er dient dazu, das Ende des Schafts 12 abzuwinkeln, das durch einen steifen Rohrabschnitt 20 gebildet sein kann. So kann das distale Ende des Schafts von einer geraden (gestreckten) Form in eine Bogenform überführt werden, die sich um eine Krümmungsachse 21 krümmt, die quer zu dem Schaft 2 orientiert ist und außerhalb desselben verläuft.

Der flexible Abschnitt 19 kann, wie in Figur 4 schematisch angedeutet ist, an einander diametral gegenüber liegenden Seiten unterschiedliche axiale Steifigkeiten haben. Beispielsweise kann er an der der Krümmungsachse 21 zugewandten Seite einen gut komprimierbaren Bereich 22 aufweisen, während an einer gegenüberliegenden Seite ein weniger gut längs komponierbarer Bereich 23 ausgebildet ist. In dieser Gestaltung bildet der flexible Abschnitt 19 eine Abwinkelvorrichtung 24. Diese kann jedoch auch auf jede andere Weise gebildet sein, die ein gezieltes Überführen des distalen Endes des Schafts von einer gestreckten Form in eine abgewinkelte Form gestattet.

Zur Betätigung der Abwinkelvorrichtung 24 ist in oder an dem mit einem Griff 25 versehenen Gehäuse 15 eine Betätigungseinrichtung 26, beispielsweise in Gestalt eines in oder an dem Gehäuse 15 schwenkbar gelagerten Hebels 27, vorgesehen. Dieser Hebel oder eine sonstige Handhabe kann über ein geeignetes Getriebe, zu dem ein Kraftübertragungsmittel 28, beispielsweise in Gestalt eines Zugmittels 29, gehört, eine Betätigungskraft der Betätigungseinrichtung 26 auf die Abwinkeleinrichtung 24 übertragen. Beispielsweise kann das Zugmittel 29 dazu direkt oder über ein geeignetes Getriebe proximal mit dem Hebel 27 und distal mit dem Endabschnitt 20 verbunden sein, wobei es sich von dem Gehäuse 15 durch den Schaft 12 bis zu dem distalen Ende 16 des Schafts 12 erstreckt. Wird es gespannt, komprimiert es den Bereich 22 axial, weswegen sich der flexible Abschnitt 19 seitlich krümmt.

Das Gehäuse 15 ist außerdem über ein Kabel 30 mit einem Stecker 31 verbunden, der zum Anschluss des Sondenapplikators 10 an ein speisendes Gerät dient. Dieses ist dazu eingerichtet, die Sonde 11 mit dem nötigen Betriebsmedium, beispielsweise elektrischer Spannung und/oder einem flüssigen oder gasförmigen Fluid oder auch Vakuum zu versorgen. Außerdem kann der Stecker 31 dazu eingerichtet sein, Steuersignale von dem Sondenapplikator 10, insbesondere von an diesen vorgesehenen Steuerelementen 32, 33, an das versorgende Gerät zu übertragen. Die Steuerelemente 32, 33 können beispielsweise elektrische Schalter sein, deren Schaltzustand über Leitungen 34, 35 (Figur 5), die sich durch das Kabel 30 erstrecken, zu entsprechenden Pins 36, 37 an dem Stecker 31 gegeben werden. Beispielsweise kann der Schalter 32 den Stromfluss über eine Diode in einer Stromrichtung zwischen den beiden Leitungen 34, 35 freigeben, während der Schalter 33 den Stromfluss über eine andere anders herum gepolte Diode in Gegenrichtung zwischen den beiden Leitungen 34, 35 freigibt. Auf diese Weise kann an den beiden Pins 36, 37 unterschieden werden, ob kein Schalter, ein Schalter oder beide Schalter betätigt sind. Andere Verschaltungen sind möglich, z.B. können alle Schalter an eine gemeinsame Bezugspotentialleitung und eine individuelle Signalleitung angeschlossen sein.

Anstelle der elektrischen Leitungen 34, 35 können auch ein oder mehrere andere Signalleitungen, beispielsweise optische Wellenleiter oder dergleichen, vorgesehen sein, wobei wenigstens ein Steuerelement 32 und/oder 33 dann zum Beispiel die Reflexionseigenschaften am Ende eines solchen optischen Wellenleiters beeinflusst um eine Signalgabe zu bewirken.

Der Stecker 31 enthält eine Anschlussvorrichtung 38 für die Sonde 11. Die Anschlussvorrichtung 38 kann beispielsweise eine in dem Stecker 31 ausgebildete Öffnung 39 sein, in die ein an dem proximalen Ende der Sonde 11 vorgesehener Sondenstecker 40 eingesteckt werden kann. Dieser dient zur Versorgung der Sonde 11 mit ihrem Betriebsmedium. Zum Beispiel kann der Sondenstecker 40, wie in Figur 5 in einer ersten Variante dargestellt ist, einen elektrischen Kontakt 41 aufweisen, dem eine in der Öffnung 39 vorgesehene Kontaktbuchse 42 oder andere Kontaktmittel zugeordnet ist oder sind. Diese Kontaktbuchse 42 ist mit einem entsprechenden Pin 43 des Steckers 31 elektrisch verbunden.

Die Sonde 11 kann, wie insbesondere aus Figur 1 und 2 hervorgeht, über geeignete Klammern 44 bis 47 oder durch andere geeignete Mittel mit dem Kabel 30 verbunden sein. Die Sonde erstreckt sich dann, ausgehend von der Anschlussvorrichtung 38, durch das Gehäuse 15 und den Schaft 12 hindurch bis zu dem distalen Ende 20 des Schafts 12. Wie Figur 3 erkennen lässt, kann in oder an dem Gehäuse 15 eine Feststelleinrichtung 48 vorgesehen sein, die manuell betätigbar ist, um eine axiale Bewegung der Sonde 11 wahlweise freizugeben oder zu sperren. Die Feststelleinrichtung 48 kann beispielsweise eine Klemmeinrichtung sein, zum Beispiel in Gestalt eines Exzenterklemmhebels 49, dem ein Widerlager 50 gegenüber liegt. Die Sonde 11 ist zwischen dem Widerlager 50 und dem Klemmhebel 49 angeordnet und kann durch Betätigung des Klemmhebels 49 entsprechend fixiert werden.

Der insoweit beschriebene Sondenapplikator 10 arbeitet wie folgt:

Zur Verwendung wird eine Sonde 11 durch eine entsprechende Einführöffnung 51 (Figur 3), die dem proximalen Ende 13 des Schafts 12 gegenüber liegt und in gerader Verlängerung desselben angeordnet ist, in das Gehäuse 15 ein- und durch dieses hindurch in das Lumen 17 eingeschoben. Die Sonde 11 wird dabei zunächst vorzugsweise so weit vorgeschoben, dass ihr distales Ende 18 aus dem Endabschnitt 20 herausragt oder kurz vor dessen Mündung steht. In dieser Position kann die Sonde 11 mit der Feststelleinrichtung 48 fixiert werden. Vor oder nach dieser Maßnahme wird der Sondenstecker 40 in die Anschlussvorrichtung 38 eingeführt und der Stecker 31 wird mit dem versorgenden Gerät verbunden. Gegebenenfalls wird an dem Gerät die gewünschte Betriebsart gewählt. Damit ist der Sondenapplikator 10 mit der eingefügten Sonde 11 betriebsbereit.

Der Schaft 12 kann mit seinem distalen Ende 16 durch eine Öffnung, zum Beispiel einen Schnitt, in einen Patienten eingeführt werden, wobei der Sondenapplikator 10 von dem Behandler an dem Griff 25 gehalten und mittels dessen positioniert wird. Falls gewünscht, kann der Behandler mittels der Betätigungseinrichtung 26 das distale Ende 16 des Schafts 12 abwinkeln, um das distale Ende 18 der Sonde 11 in eine gewünschte Winkellage zu bringen. Außerdem kann er den Schaft 12 insbesondere bei abgewinkeltem Ende um seine Längsachse drehen. Falls gewünscht, kann er außerdem die Klemmvorrichtung 48 lösen und die Sonde 11 weiter vorschieben oder zurückziehen. Mit den Steuerelementen 32, 33 kann er das speisende Gerät veranlassen, die Sonde 11 zu aktivieren oder zu deaktivieren. Beispielsweise kann er über die Steuerelemente 32, 33 Strom einschalten, Strom ausschalten oder verschiedene Moden zum Beispiel Spannungshöhen oder Spannungsformen (Kurvenformen, Crestfaktoren) auswählen und aktivieren sowie deaktivieren.

Die Anschlussvorrichtung 38 ist nicht auf Versorgung elektrischer Sonden beschränkt. Sie kann zum Beispiel zusätzlich oder alternativ zu dem Kontaktmittel 32 einen Fluidanschluss 52 aufweisen, zum Beispiel in Form einer Fluidsteckbuchse. Diese kann über einen geeigneten Kanal mit einem Fluidsteckpin 53 verbunden sein, der vorzugsweise parallel zu den Kontaktpins 36, 37, 43 von dem Stecker 31 weg ragt. Entsprechend kann eine Sonde lla mit einem Fluidsteckpin 54 versehen sein, der dem Fluidanschluss 52 zugeordnet ist. Zusätzlich kann wiederum ein elektrischer Kontakt 41 vorgesehen sein. In diesem Fall können die Steuerelemente 32, 33 dazu dienen, die Fluidversorgung und die Stromversorgung der Sonde lla voneinander unabhängig zu aktivieren oder zu deaktivieren.

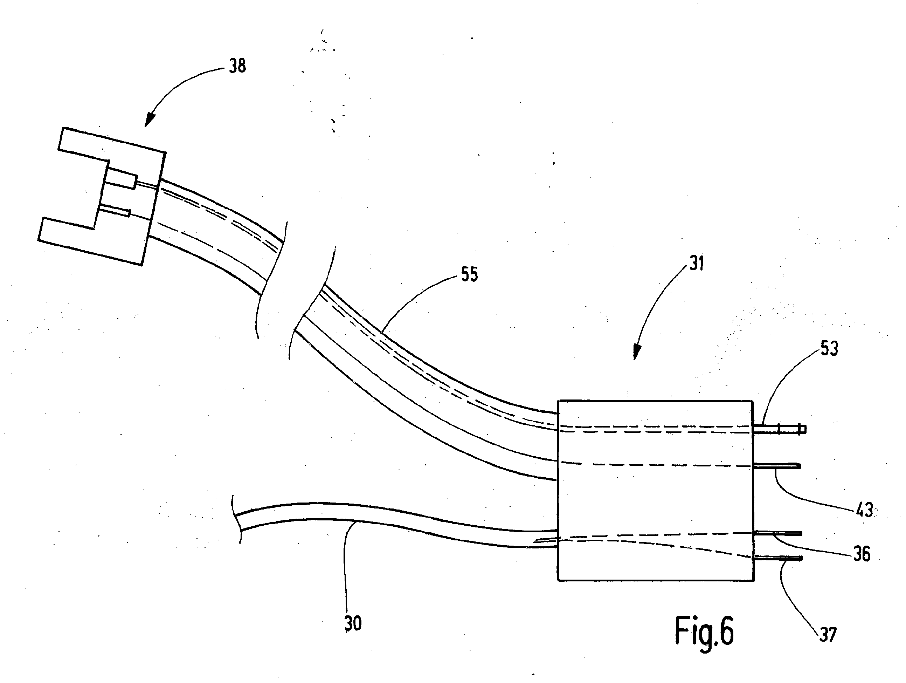

Wie aus Figur 6 ersichtlich ist, muss die Anschlussvorrichtung 38 nicht zwingend unmittelbar an dem Stecker 31 ausgebildet sein. Es ist auch möglich, als Anschlussvorrichtung 38 eine gesonderte Buchsenanordnung vorzusehen, die über eine Leitung 55 mit dem Stecker 31 verbunden ist. Weiter alternativ kann die Anschlussvorrichtung 38 an dem speisenden Gerät selbst vorgesehen sein. Ansonsten gilt die vorige Beschreibung entsprechend.

Erfindungsgemäß ist ein Sondenapplikator 10 vorgesehen, der zur Aufnahme flexibler Sonden 11, lla dient, die für den endoskopischen Einsatz vorgesehen sind und mit dem Sondenapplikator für den laparoskopischen Einsatz zugänglich gemacht werden. Die Steuerung des die Sonden 11, lla speisenden Geräts erfolgt über Steuerelemente 32, 33 des Sondenapplikators 10. Vorzugsweise erfolgt die Versorgung der Sonde 11, lla über eine an dem Sondenapplikator 10 vorgesehene Anschlussvorrichtung 38.

**Bezugszeichen:**

| | |
|---|---|
| 10 | Sondenapplikator |
| 11 | Sonde |
| 12 | Schaft |
| 13 | proximales Ende des Schafts 12 |
| 14 | Schaftaufnahme |
| 15 | Gehäuse |
| 16 | distales Ende des Schafts 12 |
| 17 | Lumen |
| 18 | distales Ende der Sonde 11 |
| 19 | flexibler Abschnitt des Schafts 12 |
| 20 | Endabschnitt des Schafts 12 |
| 21 | Krümmungsachse |
| 22 | axial nachgiebiger Bereich |
| 23 | axial steifer Bereich |
| 24 | Abwinkeleinrichtung |
| 25 | Griff |
| 26 | Betätigungseinrichtung |
| 27 | Hebel |
| 28 | Kraftübertragungsmittel |
| 29 | Zugmittel |
| 30 | Kabel |
| 31 | Stecker |
| 32, 33 | Steuerelemente |
| 34, 35 | Leitungen |
| 36, 37 | Pins |
| 38 | Anschlussvorrichtung |
| 39 | Öffnung |
| 40 | Sondenstecker |
| 41 | elektrischer Kontakt |
| 42 | Kontaktmittel |
| 43 | Pin des Steckers 31 |
| 44 - 47 | Klammern |
| 48 | Feststelleinrichtung |
| 49 | Exzenterklemmhebel |
| 50 | Widerlager |
| 51 | Einführöffnung |
| 52 | Fluidanschluss |
| 53 | Fluidsteckpin |
| 54 | Fluidsteckpin |
| 55 | Leitung |

## Patentansprüche

1. Sondenapplikator (10) zum laparoskopischen Einsatz von flexiblen Sonden (11, 11a),
mit einem Gehäuse (15), das einen Griff (25) zur manuellen Positionierung und Bewegung des Sondenapplikators (10) in Bezug auf einen Patienten aufweist,
mit einem Schaft (12), der ein längliches, steifes Rohr ist, das an einem proximalen Ende (13) mit einer an dem Gehäuse (15) vorgesehenen Schaftaufnahme (14) verbunden und an seinem distalen Ende (16) offen ist, mit wenigstens einem an dem Gehäuse (15) zur manuellen Betätigung zugänglich angeordneten Steuerelement (32, 33),
mit einem Kabel (30), das sich von dem Gehäuse (15) zu einem Stecker (31) mit wenigstens einem Steckverbinder (36) erstreckt und wenigstens eine Signalleitung (34) enthält, die das Steuerelement (32) mit dem Steckkontakt (36) verbindet,
mit einer an dem Gehäuse (15) vorgesehenen Sonden-Einführöffnung (51), die der Schaftaufnahme (14) gegenüberliegend angeordnet ist, und
mit einer Anschlussvorrichtung (38), die zum Anschluss einer Sonde (11, 11a) eingerichtet ist, um diese mit Betriebsmedium zu versorgen,
**dadurch gekennzeichnet, dass** die Anschlussvorrichtung (38) an dem Stecker (31) angeordnet ist.

2. Sondenapplikator nach Anspruch 1, **dadurch gekennzeichnet, dass** der Schaft (12) eine Länge aufweist, die das Zwanzigfache, vorzugsweise das Fünfzig- bis Neunzigfache des Durchmessers des Schafts (12) übersteigt und vorzugsweise geringer als das 300-fache, weiter vorzugsweise geringer als das 150-fache des Durchmessers des Schafts (12) ist.

3. Sondenapplikator nach einem der vorstehenden Ansprüche, **dadurch gekennzeichnet, dass** an dem distalen Ende (16) des Schafts (12) eine Abwinkelungseinrichtung (24) angeordnet ist, die mit einer an dem Gehäuse (15) angeordneten Betätigungseinrichtung (26) verbunden ist.

4. Sondenapplikator nach Anspruch 3, **dadurch gekennzeichnet, dass** die Betätigungseinrichtung (26) ein an dem Griff (25) angeordneter Schwenkhebel (27) ist, der über ein Kraftübertragungsmittel (28) mit der Abwinkelungseinrichtung (24) verbunden ist.

5. Sondenapplikator nach einem der vorstehenden Ansprüche, **dadurch gekennzeichnet, dass** die Abwinkelungseinrichtung (24) einen flexiblen Abschnitt (19) aufweist, der von einer gestreckten Form in eine Bogenform überführbar ist.

6. Sondenapplikator nach einem der vorstehenden Ansprüche, **dadurch gekennzeichnet, dass** die Abwinkelungseinrichtung (24) an ihrem distalen Ende einen steifen geraden Rohrabschnitt (20) trägt.

7. Sondenapplikator nach einem der vorstehenden Ansprüche, **dadurch gekennzeichnet, dass** der Schaft (12) an dem Gehäuse (15) um eine Schaftlängsachse drehbar gehalten ist.

8. Sondenapplikator nach einem der vorstehenden Ansprüche, **dadurch gekennzeichnet, dass** an der Sonden-Einführöffnung (51) eine Feststelleinrichtung (48) zur axialen Fixierung einer sich durch die Sonden-Einführöffnung (51) erstreckenden Sonde (11, 11a) angeordnet ist.

9. Sondenapplikator nach Anspruch 8, **dadurch gekennzeichnet, dass** die Feststelleinrichtung (48) eine manuell betätigbare Klemmeinrichtung (49) ist.

10. Sondenapplikator nach einem der vorstehenden Ansprüche, **dadurch gekennzeichnet, dass** die Signalleitung (34) eine elektrische Leitung ist und dass der Steckverbinder ein elektrischer Steckkontakt (36) ist.

11. Sondenapplikator nach einem der vorstehenden Ansprüche, **dadurch gekennzeichnet, dass** das Kabel (30) Verbindungsmittel (44 - 47) zur Aufnahme einer Sonde (11, 11a) trägt.

12. Sondenapplikator nach einem der vorstehenden Ansprüche, **dadurch gekennzeichnet, dass** die Anschlussvorrichtung (38) wenigstens einen Fluidanschluss (52) umfasst.

13. Sondenapplikator nach einem der vorstehenden Ansprüche, **dadurch gekennzeichnet, dass** die Anschlussvorrichtung (38) wenigstens einen elektrischen Anschluss (42) umfasst.

## Claims

1. Probe applicator (10) for laparoscopic use of flexible probes (11, 11a),
with a housing (15) which has a handle (25) for manual positioning and movement of the probe applicator (10) with respect to a patient,
with a shaft (12) which is an elongate stiff tube that is connected at a proximal end (13) to a shaft receiver (14) provided on the housing (15) and is open at its distal end (16),
with at least one control element (32, 33) arranged on the housing (15) so as to be accessible for manual actuation,
with a cable (30) which extends from the housing (15) to a connector (31) with at least one plug connector (36), and contains at least one signal line (34) that connects the control element (32) to the plug contact (36),
with a probe insertion opening (51) which is provided on the housing (15) and arranged opposite the shaft receiver (14), and
with an attachment device (38) which is configured for attachment of a probe (11, 11a) in order to supply this with operating medium,
**characterised in that** the attachment device (38) is arranged on the connector (31).

2. Probe applicator according to claim 1, **characterised in that** the shaft (12) has a length which exceeds twenty times, preferably fifty to ninety times the diameter of the shaft (12), and is preferably less than 300 times, further preferably less than 150 times the diameter of the shaft (12).

3. Probe applicator according to any of the preceding claims, **characterised in that** a bending device (24) is arranged at the distal end (16) of the shaft (12) and is connected to an actuating device (26) arranged on the housing (15).

4. Probe applicator according to claim 3, **characterised in that** the actuating device (26) is a pivot lever (27) which is arranged on the handle (25) and is connected to the bending device (24) via a force-transmission means (28).

5. Probe applicator according to any of the preceding claims, **characterised in that** the bending device (24) has a flexible portion (19) which can be transferred from an elongate form into a curved form.

6. Probe applicator according to any of the preceding claims, **characterised in that** at its distal end, the bending device (24) carries a stiff, straight tube portion (20).

7. Probe applicator according to any of the preceding claims, **characterised in that** the shaft (12) is held on the housing (15) so as to be rotatable about a shaft longitudinal axis.

8. Probe applicator according to any of the preceding claims, **characterised in that** at the probe insertion opening (51), a fixing device (48) is arranged for axially fixing a probe (11, 11a) extending through the probe insertion opening (51).

9. Probe applicator according to claim 8, **characterised in that** the fixing device (48) is a manually actuatable clamping device (49).

10. Probe applicator according to any of the preceding claims, **characterised in that** the signal line (34) is an electrical line, and the plug connector is an electrical plug contact (36).

11. Probe applicator according to any of the preceding claims, **characterised in that** the cable (30) carries connecting means (44 - 47) for receiving a probe (11, 11a).

12. Probe applicator according to any of the preceding claims, **characterised in that** the attachment device (38) comprises at least one fluid connection (52).

13. Probe applicator according to any of the preceding claims, **characterised in that** the attachment device (38) comprises at least one electrical connection (42).

## Revendications

1. Applicateur de sonde (10) destiné à l'utilisation laparosco-pique de sondes (11, 11a) souples,
comprenant un boîtier (15) qui présente une poignée (25) pour le positionnement et le déplacement manuels de l'applicateur de sonde (10) par rapport à un patient,
comprenant une tige (12) qui est un tube rigide allongé, lequel est relié par une extrémité proximale (13) à un raccord de tige (14), prévu sur le boîtier (15), et est ouvert à son extrémité distale (16),
comprenant au moins un élément de commande (32, 33) disposé de manière accessible sur le boîtier (15) en vue de l'actionnement manuel,
comprenant un câble (30) qui s'étend du boîtier (15) jusqu'à un élément de connexion (31), comportant au moins un connecteur enfichable (36), et contient au moins une ligne de transmission de signaux (34) qui relie l'élément de commande (32) au contact enfichable (36),
comprenant une ouverture d'insertion de sonde (51) qui est prévue sur le boîtier (15) et est disposée en vis-à-vis du raccord de tige (14), et
comprenant un dispositif de raccordement (38) qui est conçu pour le raccordement d'une sonde (11, 11a) afin d'alimenter celle-ci avec un fluide de travail,
**caractérisé en ce que** le dispositif de raccordement (38) est placé sur l'élément de connexion (31).

2. Applicateur de sonde selon la revendication 1, **caractérisé en ce que** la tige (12) présente une longueur qui est supérieure de vingt fois, de préférence de cinquante à quatre-vingt-dix fois le diamètre de la tige (12) et est de préférence inférieure à 300 fois, de préférence inférieure à 150 fois le diamètre de la tige (12).

3. Applicateur de sonde selon l'une des revendications précédentes, **caractérisé en ce qu'**il est prévu à l'extrémité distale (16) de la tige (12), un dispositif de cintrage (24) qui est relié à un dispositif d'actionnement (26) placé sur le boîtier (15).

4. Applicateur de sonde selon la revendication 3, **caractérisé en ce que** le dispositif d'actionnement (26) est un levier pivotant (27) qui est disposé sur la poignée (25) et est relié au dispositif de cintrage (24) par l'intermédiaire d'un moyen de transmission de force (28).

5. Applicateur de sonde selon l'une des revendications précédentes, **caractérisé en ce que** le dispositif de cintrage (24) présente une partie souple (19) qui peut être amenée d'une forme allongée à une forme incurvée.

6. Applicateur de sonde selon l'une des revendications précédentes, **caractérisé en ce que** le dispositif de cintrage (24) porte à son extrémité distale une portion tubulaire (20) rectiligne rigide.

7. Applicateur de sonde selon l'une des revendications précédentes, **caractérisé en ce que** la tige (12) est tenue sur le boîtier (15) avec possibilité de rotation autour d'un axe longitudinal de tige.

8. Applicateur de sonde selon l'une des revendications précédentes, **caractérisé en ce qu'**il est prévu sur l'ouverture d'insertion de sonde (51), un dispositif de blocage (48) destiné à la fixation axiale d'une sonde (11, 11a) s'étendant à travers l'ouverture d'insertion de sonde (51).

9. Applicateur de sonde selon la revendication 8, **caractérisé en ce que** le dispositif de blocage (48) est un dispositif de serrage (49) à actionnement manuel.

10. Applicateur de sonde selon l'une des revendications précédentes, **caractérisé en ce que** la ligne de transmission de signaux (34) est une ligne électrique et **en ce que** le connecteur enfichable est un contact enfichable électrique (36).

11. Applicateur de sonde selon l'une des revendications précédentes, **caractérisé en ce que** le câble (30) porte des moyens de liaison (44 à 47) destinés à recevoir une sonde (11, 11a).

12. Applicateur de sonde selon l'une des revendications précédentes, **caractérisé en ce que** le dispositif de raccordement (38) comprend au moins un raccord de fluide (52).

13. Applicateur de sonde selon l'une des revendications précédentes, **caractérisé en ce que** le dispositif de raccordement (38) comprend au moins une connexion électrique (42).
